# EUROPÄISCHE PATENTANMELDUNG

(11) **EP 2 363 157 A1**
(43) Veröffentlichungstag der Anmeldung: **07.09.2011**
(21) Anmeldenummer: 10075103.1
(22) Anmeldetag: 05.03.2010
(51) Int. Cl.: A61M 1/10, F04D 29/38

(54) **Vorrichtung zur mechanischen Einwirkung auf ein Medium, insbesondere Fluidpumpe**

(71) Anmelder: ECP Entwicklungsgesellschaft mbH, 12247 Berlin (DE)
(72) Erfinder: Wiessler, Petra, 13086 Berlin (DE); Friedel, Sven-René, 10555 Berlin (DE); Liebing, Reiner, 14469 Potsdam (DE); Er, Sami, 10715 Berlin (DE); Schlicht, Henning, 14471 Potsdam (DE)
(74) Vertreter: Pfenning, Meinig & Partner GbR

(57) **Zusammenfassung**

Die Erfindung bezieht sich auf eine Vorrichtung zur mechanischen Einwirkung auf ein Medium (30) mit wenigstens einem ersten Element (9''', 10'''), das durch Änderung wenigstens einer mechanischen Eigenschaft von einem Transportzustand in einen Betriebszustand gebracht werden kann.

Eine solche Vorrichtung kann beispielsweise eine Blutpumpe für den medizinischen, mikroinvasiven Bereich sein.

Die Aufgabe, einen möglichst komfortablen Übergang zwischen dem Transportzustand und dem Betriebszustand zu erreichen und dabei möglichst große Freiheit bei der Konstruktion der entsprechenden Vorrichtung, insbesondere einer Pumpe, zu lassen, wird mit den Mitteln der Erfindung dadurch gelöst, dass das erste Element wenigstens teilweise aus einem Stoff (24, 25, 26, 27) besteht oder mit einem Stoff oder Stoffgemisch füllbar ist, der/das zum Übergang in den Betriebszustand eine Stoffumwandlung durch eine chemische Reaktion, insbesondere Vernetzung, oder eine Kristallisation durchläuft.

## Beschreibung

Die Erfindung liegt auf dem Gebiet des Maschinenbaus und der Feinwerktechnik und ist mit besonderem Vorteil bei Vorrichtungen einsetzbar, die in einem Transportzustand an einen Einsatzort befördert und dort in einen Betriebszustand gebracht werden, bevor sie in Gang gesetzt werden.

Dies ist beispielsweise bei Geräten sinnvoll, die an schwierig zugängliche Orte befördert werden müssen, um dort eingesetzt zu werden, beispielsweise Pumpen oder Bearbeitungswerkzeuge in verwinkelten Schlauch-oder Rohrsystemen.

Im mikroskopischen Maßstab sind derartige Geräte als mikroinvasive Geräte, beispielsweise Pumpen oder Fräsen in menschlichen oder tierischen Gefäßen, beispielsweise Blutgefäßen oder anderen Körperhohlräumen einsetzbar.

Dabei ist es schwierig, diese Geräte durch die körpereigenen Gefäße einzuführen, da hierzu die entsprechenden Maße sehr klein gehalten werden müssen, wobei gleichzeitig im Betrieb größere Abmaße für die Effektivität des Einsatzes sinnvoll sind.

Auf dem Gebiet der Katheterpumpen, insbesondere der Blutpumpen, sind zur Lösung dieses Problems bereits radial komprimierbare Pumpen vorgeschlagen worden, die während des Transports in einem Transportzustand mit geringer radialer Ausdehnung gehalten werden und die nach dem Einbringen an den Einsatzort, beispielsweise in eine Herzkammer, dort radial expandiert werden können.

Zu diesem Zweck sind aufwendige mechanische Konstruktionen bekannt, die zum Aufrichten von Förderelementen eines Rotors dienen. Zudem ist es oft notwendig, die entsprechenden Förderelemente wie Förderschaufeln im Betrieb zu stabilisieren, da diese beträchtlichen Fluidkräften im Betrieb ausgesetzt sind.

Beispielsweise ist ein komprimierbarer Rotor aus der US 6 860 713 bekannt. Zudem ist aus der US 7 393 181 B2 ein weiterer Rotor bekannt. Bei den aus der Patentliteratur bekannten Lösungen ist teilweise vorgesehen, dass ein Rotor durch elastisch verformbare Förderschaufeln komprimierbar ist oder dass Aufrichtmechanismen für Förderschaufeln geschaffen werden, die ansonsten bei ruhendem Rotor an diesen angelegt sind.

Zusätzlich zur Komprimierbarkeit des Rotors kann es auch vorteilhaft oder sinnvoll sein, das Pumpengehäuse, das den Rotor umgeben kann, entsprechend komprimierbar auszugestalten.

Dabei besteht regelmäßig das Problem, dass einerseits die Konstruktion und die Materialien des Rotors im Betrieb stabil sein sollen, um das Fluid bei hohen Drehzahlen zuverlässig zu fördern, und dass andererseits eine gewisse Nachgiebigkeit zumindest von Teilen des Rotors wünschenswert ist, um die zur Kompression des Rotors bzw. der Pumpe notwendigen Kräfte in Grenzen zu halten.

Der vorliegenden Erfindung liegt vor diesem Hintergrund die Aufgabe zugrunde, eine entsprechende Vorrichtung zur mechanischen Einwirkung auf ein Medium, insbesondere eine Fluidpumpe, derart zu gestalten, dass sie sinnvoll von einem Transportzustand in einen Betriebszustand gebracht werden kann, wobei der Transportzustand eine besondere Eignung für den Transport aufweist, während der Betriebszustand sich von diesem unterscheidet und besonders für den Betrieb der Vorrichtung/Pumpe geeignet ist.

Die Aufgabe wird mit den Merkmalen der Erfindung gemäß Patentanspruch 1 gelöst.

Dadurch, dass das erste Element aus einem Stoff besteht bzw. mit einem Stoff oder Stoffgemisch gefüllt oder füllbar ist, der/das beim Übergang in den Betriebszustand eine chemische Reaktion, beispielsweise eine Vernetzung oder einen Übergang vom flüssigen Zustand in einen festen Zustand durchläuft und damit wenigstens eine mechanische Eigenschaft ändert, ist es möglich, beispielsweise im Transportzustand des ersten Elementes eine geringere Steifigkeit zu realisieren als im Betriebszustand. Beispielsweise kann das erste Element ein Förderelement, beispielsweise eine Förderschaufel eines Rotors einer Fluidpumpe sein, so dass bei geringerer Steifigkeit das entsprechende Förderelement an eine Nabe anlegbar ist und der entsprechende Rotor damit leicht komprimierbar ist. Der Rotor kann in diesem Zustand auch selbstkomprimierbar sein, dadurch, dass im Ruhezustand die Förderelemente an der Nabe anliegen und diese erst durch Zentrifugalkräfte bei Inbetriebnahme aufgestellt werden.

Um dann im Betrieb eine höhere Steifigkeit der Förderschaufeln oder Förderelemente zu gewährleisten, ist der Stoff, aus dem die Förderschaufel oder ein Teil der Förderschaufel besteht, derart gewählt, dass er bei Durchlaufen der entsprechenden Reaktion oder des Übergangs, insbesondere des Übergangs in einen anderen Aggregatzustand, beispielsweise einer Kristallisation, eine Erhärtung oder Versteifung erfährt.

Es kann auch vorgesehen sein, dass das erste Element, also beispielsweise das Förderelement, Hohlräume aufweist, die mit einem entsprechenden Stoff füllbar sind, beispielsweise über Zuleitungsschläuche, und die danach härtbar, versteifbar oder, wenn es sich um eine Flüssigkeit handelt, in ihrer Viskosität änderbar sind.

Dabei muss nicht das gesamte Element seine Eigenschaften entsprechend ändern, sondern es genügt beispielsweise, wenn ein Gelenk, das das Förderelement mit einer Nabe verbindet, durch die entsprechende Reaktion versteift wird oder seine Form ändert und dadurch das Förderelement radial expandiert bzw. aufrichtet.

Es kann auch vorgesehen sein, dass das erste Element ein anderes Teil eines Rotors einer Fluidpumpe darstellt oder ein Pumpengehäuse entsprechend in eine Betriebsform oder eine dem Betrieb entsprechende Steifigkeit zu bringen ist.

Auch in diesem Fall wie bei einer entsprechenden Anwendung bei einem Rotor können Hohlräume vorgesehen sein, die beispielsweise unter Druck mit einem Fluid gefüllt und damit aufgepumpt werden können, um dem entsprechenden ersten Element eine gewünschte Form zu geben, woraufhin der Stoff gehärtet oder versteift werden kann, um diese Form stabil zu erhalten.

Durch die entsprechende Reaktion kann alternativ zur Steifigkeit auch eine andere mechanische Eigenschaft wie die geometrische Form oder Größe geändert werden. Beispielsweise kann ein Teil einer Förderschaufel durch die Reaktion verkürzt oder verlängert werden und dadurch, also mittels Hebelkräften, die Förderschaufel aufrichten oder versteifen.

Der Stoff, aus dem das erste Element teilweise besteht oder mit dem es füllbar ist, kann beispielsweise ein härtbares Material, insbesondere einen härtbaren Kunststoff aufweisen.

Eine Härtung kann zum Übergang in den Betriebszustand beispielsweise durch Temperatur- bzw. Druckeinwirkung, elektrische und/oder magnetische Felder bzw. Impulse, Strahlung (IR- bzw. UV-Licht-, α-, β-, γ-), mechanische Einwirkung, z. B. Ultraschall oder Erschütterung erfolgen, oder auch durch Kontakt mit einem weiteren Stoff oder durch Initialisierung einer Kristallisation bei einem flüssigen Stoff herbeigeführt werden.

Der weitere Stoff kann dabei ein echter Reaktionspartner sein, der bei der Reaktion ebenfalls reagiert und eine Umwandlung erfährt, oder ein Katalysator bzw. Enzym, der bei Zugabe zu einer Beschleunigung der Reaktion führt.

Der weitere Stoff kann beispielsweise in dem Medium enthalten sein, auf das die Vorrichtung einwirken soll, beispielsweise in der Körperflüssigkeit, in der eine entsprechende Fluidpumpe betrieben werden soll. In diesem Fall kann die Körperflüssigkeit beim Einbringen der Vorrichtung in einen Körper eindiffundieren, und die gewünschte Reaktion kann dann vor Aufnahme des Betriebs, entweder selbsttätig oder initiiert oder unterstützt durch zusätzliche Maßnahmen, ablaufen.

Durch die Erfindung gelingt es, für den Betrieb der Vorrichtung, beispielsweise der Pumpe, mechanische Eigenschaften zu gewährleisten, die während des Transports zur Einsatzstelle unvorteilhaft wären und durch die Erfindung vermieden werden können. Diese Eigenschaften werden erst nach dem Verbringen an den Einsatzort mit Ablauf der entsprechenden Reaktionen erreicht. Die entsprechenden Reaktionen können reversibel, jedoch auch irreversibel sein.

Eine vorteilhafte Ausgestaltung der Erfindung sieht weiterhin vor, dass durch eine fortgesetzte oder weitere Reaktion das erste Element reversibel oder irreversibel in einen Zustand überführbar ist, in dem die Vorrichtung transportierbar ist oder in dem es durch mechanisches Zerbrechen in einen Transportzustand gebracht werden kann.

Beispielsweise kann durch Fortsetzung der Reaktion, die vor Erreichen des Betriebszustandes durchlaufen wurde, eine weitere Änderung der mechanischen Eigenschaften stattfinden, z. B. eine Versprödung des Stoffes, aus dem das erste Element ganz oder teilweise besteht. Diese Versprödung kann so weit gehen, dass die entsprechenden Teile von selbst brechen oder zumindest leicht gebrochen werden können, um wieder einen geeigneten Transportzustand zum Rücktransport der Vorrichtung/Fluidpumpe zu erreichen. Es kann jedoch auch eine andere Reaktion vorgesehen sein, um das entsprechende gewünschte Ergebnis zu erzielen.

Zusätzlich zu der erfindungsgemäßen Vorrichtung bezieht sich die Erfindung auch auf ein Verfahren zum Betrieb einer entsprechenden Vorrichtung, bei dem zunächst die Vorrichtung in einen Körper eines Lebewesens eingeführt wird, wobei darauf der Stoff / das Stoffgemisch des ersten Elementes einer Reaktion, insbesondere Vernetzung, oder eine Kristallisation aus der flüssigen Phase erfährt und wobei darauf die Vorrichtung in Gang gesetzt wird.

Durch eine derartige Handhabung wird die Vorrichtung im Transportzustand zunächst innerhalb eines Körpers eines Lebewesens an den Einsatzort gebracht und erst dort bezüglich der mechanischen Eigenschaften in den Betriebszustand und damit eine für den Betrieb effiziente Form gebracht.

Besonders vorteilhaft kann vorgesehen sein, dass vor der Reaktion der oder ein Stoff in wenigstens einen Hohlraum des ersten Elementes eingeführt wird. Beispielsweise kann ein Hohlraum oder eine Reihe von Hohlräumen, die beispielsweise durch Blasen eines Schaumstoffs, aus dem das erste Element besteht, gebildet sein können, über Schläuche mit einem Stoff gefüllt, der entweder selbst eine Reaktion zur Änderung seiner mechanischen Eigenschaften durchlaufen kann oder der in den Hohlräumen auf einen weiteren Stoff trifft, mit dem er entsprechend reagiert oder wobei einer der Stoffe als Katalysator für die Reaktion dient.

Die eigentliche Reaktion kann dabei durch entsprechende Einflussnahme von außen initiiert, unterstützt oder durchgeführt werden, wobei hierzu beispielsweise Strahlung, Temperaturveränderung, mechanische Einwirkung, wie beispielsweise Ultraschall, oder die Einwirkung durch elektrische und/oder magnetische Felder dienen kann.

Die Erfindung bezieht sich zudem auf eine Vorrichtung zur mechanischen Einwirkung auf ein Medium mit wenigstens einem ersten Element, das durch Änderung wenigstens einer mechanischen Eigenschaft von einem Transportzustand in einen Betriebszustand gebracht werden kann, wobei das erste Element wenigstens teilweise aus einem Stoff besteht oder mit einem Stoff oder Stoffgemisch füllbar ist, der/das, solange er/es einer Bestrahlung, einem elektrischen und/oder magnetischen Feld ausgesetzt ist, gegenüber dem Zustand ohne derartige Einwirkung geänderte mechanische Eigenschaften, insbesondere bezüglich Steifigkeit, Viskosität, Größe und/oder Form, aufweist.

Beispiele für eine derartige Veränderung der mechanischen Eigenschaften durch Felder sind der Piezoeffekt und Magnetostriktion bzw. bei Flüssigkeiten ein magnetorheologischer Effekt bzw. die Änderung der Viskosität durch elektrische Feldeinwirkung.

Ein entsprechendes erfindungsgemäßes Verfahren zum Betrieb einer solchen Vorrichtung sieht vor, dass die Vorrichtung nach ihrer Einführung in einen Körper eines Lebewesens der entsprechenden Einwirkung ausgesetzt wird, um die Vorrichtung funktionsfähig zu machen. Während des Betriebes der Vorrichtung/Fluidpumpe muss die Einwirkung aufrechterhalten werden. Es muss dabei weder eine reversible noch eine irreversible chemische Reaktion stattfinden, sondern es wird lediglich abhängig von der Einwirkung ein anderer mechanischer Zustand des Stoffs eingenommen.

In einer Abwandlung kann die Erfindung auch so ausgestaltet sein, dass die entsprechende Einwirkung im Transportzustand aufrechterhalten wird und zum erreichen bzw. während des Betriebszustandes entfernt wird bzw. wegfällt.

Im Folgenden wird die Erfindung anhand mehrerer Ausführungsbeispiele in einer Zeichnung gezeigt und nachfolgend beschrieben.

Dabei zeigt
- Fig. 1: schematisch in einem Längsschnitt ein Blutgefäß mit einem in dieses eingeführten Hohlkatheter und einer Fräse,
- Fig. 2: schematisch ein in eine Herzkammer mündendes Blutgefäß, durch das ein Hohlkatheter mit einer Herzpumpe eingeschoben ist,
- Fig. 3: einen Rotor einer Pumpe im Transportzustand,
- Fig. 4: den Rotor aus Fig. 3 im Betriebszustand,
- Fig. 5: einen weiteren Rotor im Transportzustand,
- Fig. 6: den weiteren Rotor aus Fig. 5 im Betriebszustand,
- Fig. 7: einen dritten Rotor im Transportzustand,
- Fig. 8: den Rotor aus Fig. 7 im Betriebszustand während des Versteifungsprozesses,
- Fig. 9: ein Detail eines Rotors mit einem Förderelement, das einen Hohlraum aufweist, der teilweise gefüllt ist,
- Fig. 10: das Detail aus Fig. 9, wobei der Hohlraum vollständig ausgefüllt ist,
- Fig. 11: schematisch eine Ansicht eines Rotors im Transportzustand mit einem Pumpengehäuse, das zusammengefallen ist,
- Fig. 12: die Pumpe aus Fig. 11 im Betriebszustand,
- Fig. 13: einen Rotor im Betriebszustand in versteifter Form mit einem im Betriebszustand befindlichen Gehäuse einer Pumpe,
- Fig. 14: die Teile der Pumpe aus Fig. 13 nach einer weiteren Behandlung, die das Brechen der Förderelemente ermöglicht,
- Fig. 15: einen Rotor mit Förderelementen, der durch einen Hohlkatheter im Transportzustand durchgeschoben wird,
- Fig. 16: den Rotor aus Fig. 15, der durch Zurückziehen der Nabe in den Hohlkatheter aufgerichtet wird,
- Fig. 17: einen Rotor, der durch Verschieben eines Stützrades mittels Schubelementen aufgerichtet wird, sowie
- Fig. 18: eine Ansicht des Schubrades.

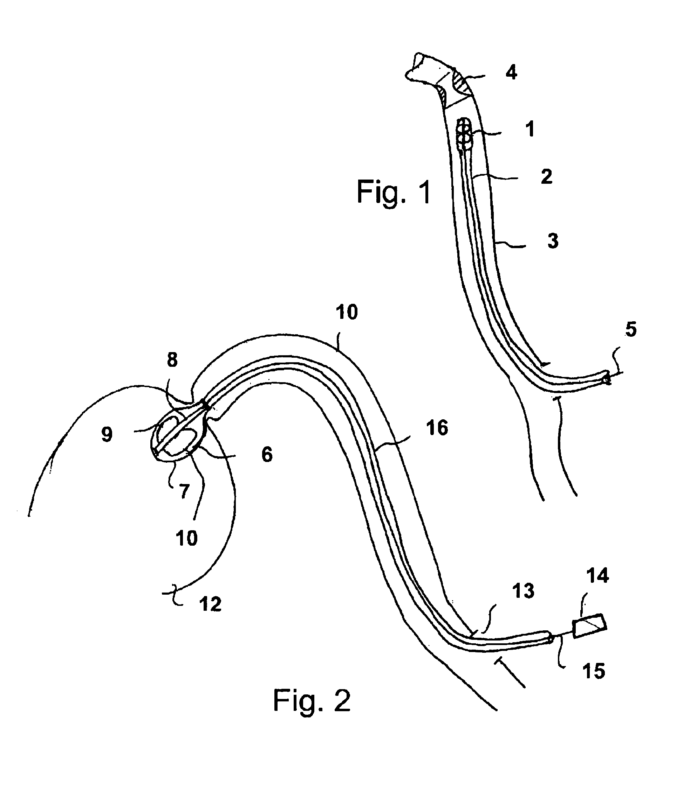

Fig. 1 zeigt als ein Beispiel für eine erfindungsgemäße Vorrichtung eine Fräse 1, die am distalen Ende eines Hohlkatheters 2 in ein Blutgefäß 3 eines menschlichen Körpers eingeführt ist, um eine Verengung 4 durch Wegfräsen von Belägen an der Wand des Blutgefäßes zu beseitigen. Innerhalb des Hohlkatheters 2 verläuft eine Welle 5, die für eine Rotation mit hoher Drehzahl ausgelegt ist und von außerhalb des Hohlkatheters durch einen Motor angetrieben werden kann.

Die Fräse 1 wird vorteilhaft zunächst in einem Transportzustand in das Blutgefäß 3 eingeführt, beispielsweise in radial komprimierter Form, und dann vor Ort in einen Betriebszustand überführt, der beispielsweise dadurch unterschiedlich zum Transportzustand sein kann, dass der Fräskopf radial vergrößert oder versteift wird. Die Erfindung löst das Problem, diese Änderung der mechanischen Eigenschaften der Fräse nach dem Durchlaufen des Transportweges in günstiger Form zu bewerkstelligen.

Fig. 2 zeigt ein weiteres Anwendungsbeispiel für eine erfindungsgemäße Vorrichtung, die in diesem Fall durch eine Herzkatheterpumpe 6 gebildet ist. Diese weist ein Gehäuse 7 auf, in dem ein Rotor mit einer Nabe 8 und Förderelementen 9, 10 in Form von Förderschaufeln untergebracht ist.

Die Pumpe weist typischerweise im Betrieb einen größeren Durchmesser auf als während des Transportes, um ihr die erforderliche Effektivität zu verleihen. Aus diesem Grund wird die Pumpe vor dem Einbringen in ein Blutgefäß 11, durch das sie in eine Herzkammer 12 geschoben werden soll, radial komprimiert. Dann wird sie durch eine Schleuse 13 in das Blutgefäß 10 eingebracht und bis zur Herzkammer 12 durchgeschoben. Darauf wird die Pumpe, beispielsweise der Rotor und das Pumpengehäuse, gemeinsam oder jeweils für sich radial expandiert. Die Erfindung kann die Expansionsbewegung an sich erzeugen oder unterstützen. Sie kann jedoch auch erst nach der Expansionsbewegung dadurch wirken, dass beispielsweise der Rotor oder das Pumpengehäuse in der expandierten Stellung versteift und damit stabilisiert wird.

Danach ist die Pumpe mit hohen Drehzahlen und unter hoher mechanischer Belastung betreibbar, indem der Motor 14 die Welle 15 mit beispielsweise 10000 U/min antreibt.

Das Aufrichten der einzelnen Elemente der Pumpe 6 nach dem Verbringen an den Einsatzort in der Herzkammer 12 kann beispielsweise dadurch geschehen, dass der Rotor 8, 9, 10 in Rotation versetzt wird und entweder durch die wirkenden Zentrifugalkräfte oder durch die Fluidgegenkräfte, die sich mit der Drehung einstellen, oder durch beides gemeinsam aufgerichtet wird. Zudem oder alternativ können auch mechanische Vorrichtungen wie beispielsweise Züge oder Druckeinrichtungen vorgesehen sein, die entlang des Hohlkatheters 16 von außerhalb des Patientenkörpers betätigbar sind und die auf den Pumpenkopf wirken und dort eine entsprechende Expansionsbewegung verursachen oder unterstützen. Es sind auch andere Mechanismen möglich, über die auf eine Expansion hingewirkt werden kann. Diese werden beispielhaft anhand der übrigen Figuren erläutert.

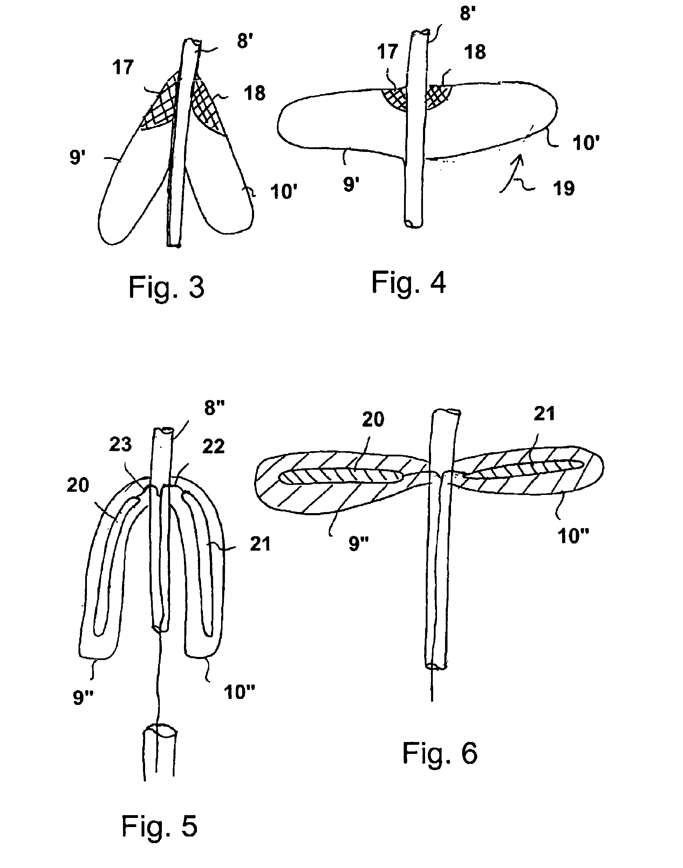

Fig. 3 zeigt eine Ansicht eines Rotors mit Förderschaufeln bzw. Rotorblättern 9', 10', die an einer Nabe 8' ansetzen und die in der Fig. 3 noch im Transportzustand gezeigt sind, in dem sie radial an die Nabe 8' angelegt sind.

Jeweils ein Teilbereich 17, 18 jedes Förderelementes 9', 10' ist derart gestaltet, dass er sich durch bestimmte äußere Einflüsse wie beispielsweise Bestrahlung mit UV-Licht oder Teilchenstrahlung (α-, β-, γ-), elektrische und/oder magnetische Felder, Ultraschall oder mechanische Belastung kontrahiert. Es können als Materialien für die Bereiche 17, 18 beispielsweise selbstvernetzende Kunststoffe gewählt werden, die bei der Vernetzung einerseits erhärten und sich andererseits zusammenziehen.

Die Fig. 4 zeigt, dass durch eine Kontraktion der Bereiche 17, 18 die Förderelemente 9', 10' in deren Bereich stärker an die Nabe 8' herangezogen und damit radial aufgerichtet werden, wie durch den Pfeil 19 angedeutet. Diese Wirkung ist bei einer dauerhaften Vernetzung stabil und dauerhaft. Es ist jedoch auch denkbar, Stoffe einzusetzen, die eine solche Kontraktion vorübergehend zeigen, beispielsweise durch Effekte der Magnetostriktion oder Piezoeffekt. In dem letztgenannten Fall wird der Rotor radial nur so lange expandiert, wie (oder bei Einwirkung nur im Transportzustand bis) die entsprechenden Felder wirken. Anderenfalls stabilisiert sich der Rotor, und die äußere Einwirkung kann wegfallen, ohne dass der Rotor instabil wird.

Es ist auch denkbar, den gesamten Bereich der Förderelemente im Bereich der Nabe aus einem entsprechenden Material herzustellen, das sich entweder kontrahiert oder versteift, wobei die Geometrie entsprechend gewählt werden muss, um eine selbsttätige Aufrichtung der Förderelemente zu erreichen, wenn nicht die Aufrichtung durch einen anderen Effekt, beispielsweise Manipulation mittels Drahtzügen oder Ähnlichem erreicht wird. Wird die Expansion durch andere Effekte erreicht, so kann es ausreichen, Teile der Förderelemente 9', 10' oder die gesamten Förderelemente zu versteifen, indem sie aus einem entsprechenden vernetzbaren Material oder einem Material, das sich unter entsprechender Einwirkung versteift, hergestellt werden. Es existieren beispielsweise Elastomere, die auf Magnetfelder durch Versteifung reagieren.

In der Fig. 5 ist eine weitere Ausführungsform der Erfindung mit einem Rotor mit einer Nabe 8'' und zwei Förderschaufeln 9'', 10'' und darin angeordneten Hohlräumen 20, 21 dargestellt.

Die Hohlräume 20, 21 sind über ein Leitungssystem mit Zuleitungen 22, 23, die durch die Nabe 8'' verlaufen, mit einer Druckquelle verbunden. Entsprechende Leitungen können entweder durch ein Lumen des Hohlkatheters oder durch zusätzlich dort innen oder außen am Hohlkatheter angeordnete Schläuche gespeist werden.

Zum Aufrichten der Förderelemente 9'', 10'' kann beispielsweise ein Gas oder eine Flüssigkeit in die Hohlräume 20, 21 eingepresst werden, so dass die Förderelemente 9'', 10'' sich, wie in Fig. 6 gezeigt, aufrichten und straffen. Eine entsprechende eingepresste Flüssigkeit in den Hohlräumen 20, 21 wird danach entweder durch Vernetzung oder eine chemische Reaktion mit einem weiteren Stoff verfestigt, oder die Eigenschaften der Flüssigkeit werden durch Feldeinwirkung geändert, was beispielsweise bei magnetorheologischen Flüssigkeiten durch Einwirkung eines magnetischen Feldes und entsprechende Änderung der Viskosität möglich ist. Damit wird der Rotor bei hoher Viskosität der Flüssigkeit stabilisiert.

Wird zunächst ein Gas eingepresst, so muss darauf ein weiterer Stoff eingebracht werden, um die Versteifung dauerhaft zu erhalten. Es können beispielsweise auch mehrere Stoffe in Form von Flüssigkeiten und/oder Gasen eingebracht werden, die entweder miteinander nach dem Zusammentreffen in den Hohlräumen 20, 21 reagieren oder die noch um einen Katalysator ergänzt werden, sobald die Förderelemente 9'', 10'' aufgerichtet sind, um die Reaktion zu beschleunigen. Wird durch die äußere Einwirkung eine irreversible Reaktion ausgelöst, so kann die Einwirkung nach der Versteifung des Rotors entfallen. Andererseits kann auch die Aufrechterhaltung beispielsweise eines Feldes notwendig sein, um die entsprechenden gewünschten mechanischen Eigenschaften des Rotors zu erhalten.

Es kann das Einpressen von Gas in die Hohlräume auch ausschließlich zum Aufrichten der Förderelemente genutzt werden, wenn danach andere Elemente des Rotors zur Stabilisierung dieses Zustands versteift werden.

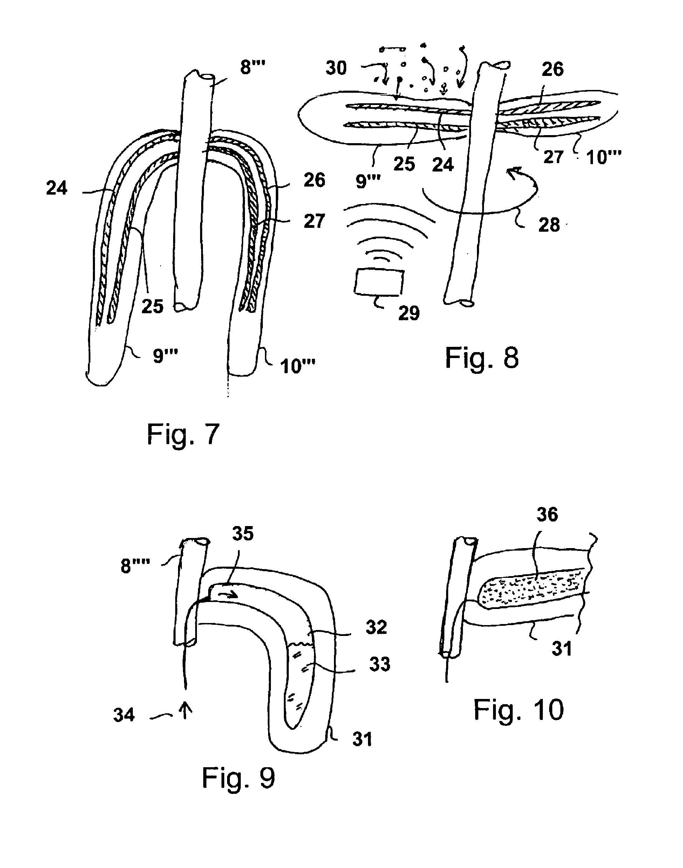

Die Fig. 7 zeigt einen Rotor mit zwei Förderelementen in Form von Schaufelblättern 9''', 10''', wobei jedes der Förderelemente zwei Versteifungsstege 24, 25, 26, 27 aufweist. Diese sind in dem Transportzustand der Fig. 7 noch schlaff, so dass die Förderelemente 9''', 10''' an der Nabe 8''' anliegen können.

Nach dem Verbringen an den Einsatzort wird, wie in Fig. 8 durch den Pfeil 28 bezeichnet, der Rotor in Drehung versetzt, so dass sich die Förderelemente 9''', 10''' durch Zentrifugalkraft und/oder Fluidgegendruck aufrichten. Zu diesem Zeitpunkt kann die Reaktion zur Versteifung der Stege 24, 25, 26, 27 einsetzen, entweder durch Bestrahlung, wie mittels der Einwirkungsquelle 29 dargestellt, die allerdings auch durch eine magnetische oder elektrische Feldquelle ersetzt werden kann, oder durch eine Ultraschallquelle oder durch eine chemische Reaktion, die beispielsweise durch Eindiffundieren eines Stoffes 30, in dem sich die Förderelemente bewegen, ausgelöst oder befördert werden kann. Dieser Stoff kann beispielweise beim Einsatz der Pumpe im menschlichen Blut als Bestandteil des Blutes in natürlicher Form vorliegen. Diffundiert dieser Stoff in die Förderelemente und trifft die Verstärkungs- oder Versteifungsstege, so findet dort eine Härtungsreaktion statt, die die Förderelemente versteift.

Die Fig. 9 zeigt schematisch ein einzelnes Förderelement 31 mit einem Hohlraum 32, der teilweise mit einer Flüssigkeit 33 gefüllt ist.

Zur Aufrichtung und/oder Versteifung des Förderelementes 31 ist vorgesehen, dass entlang der Pfeile 34, 35 ein Gas durch ein Lumen in der Nabe 8'''' und das Förderelement 31 in den Hohlraum 32 einströmt und dort mit der Flüssigkeit 33 unter Bildung eines Schaumstoffs reagiert. Hierdurch und durch die entsprechende Reaktion findet eine Expansion statt, durch die der Hohlraum 32 unter Druck gesetzt und aufgeblasen wird. Gleichzeitig versteift sich der Schaumstoff 36 entweder durch die Reaktion oder durch eine nachfolgende Härtung und stabilisiert somit das Förderelement 31, wie in der Fig. 10 dargestellt.

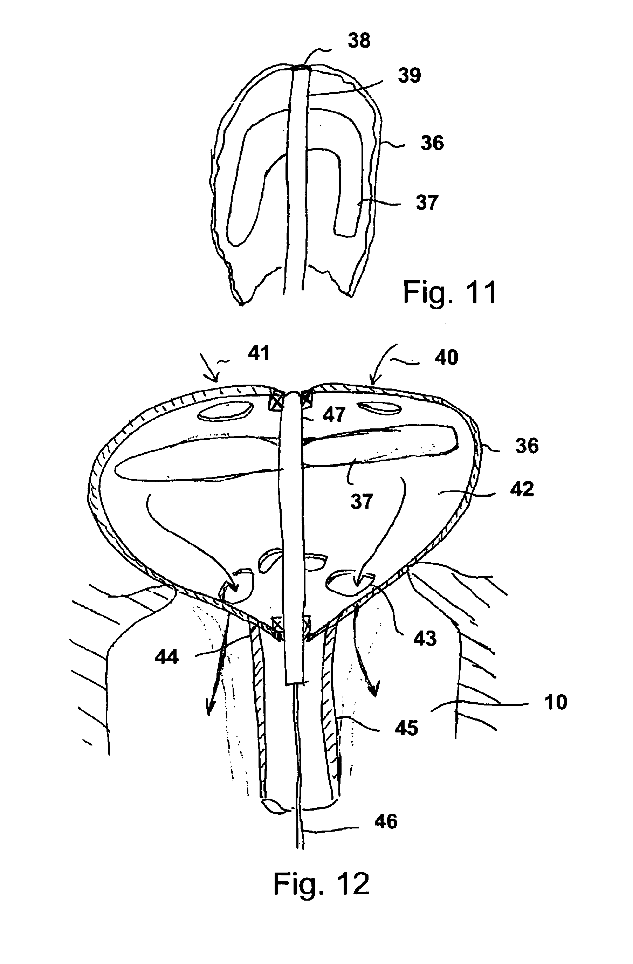

Fig. 11 zeigt im Transportzustand eine Pumpe mit einem Pumpengehäuse 36 in Form einer Membran, die zusammengefallen ist und den ebenfalls komprimierten Rotor mit den Förderschaufeln 37 eng umgibt. Das Gehäuse 36 ist am Ende 38 der Nabe 39 befestigt und wird in diesem Zustand am Ende eines Hohlkatheters durch ein Blutgefäß geschoben.

Ist der Pumpenkopf bei der Anwendung als Herzpumpe durch den Aortenbogen durch- und in eine Herzkammer eingeschoben, so kann der Rotor langsam in Drehung versetzt werden, wie in der Fig. 12 gezeigt. Durch die Zentrifugalkräfte und/oder durch Fluidgegenkräfte des zu fördernden Blutes werden die Förderschaufeln 37 aufgerichtet, saugen Blut durch Öffnungen an der Vorderseite des Gehäuses 36, angedeutet durch die Pfeile 40, 41, ein und erhöhen damit den Druck im Innenraum des Pumpengehäuses 36. Hierdurch wird die Membran 36 aufgeweitet und aufgeblasen und spannt sich straff. Gleichzeitig öffnet sich der Raum zur vollständigen Entfaltung der Förderschaufeln 37, so dass der Rotor seine volle Umdrehungszahl aufnehmen kann. Aus dem Innenraum 42 des Pumpengehäuses 36 kann dann das Blut durch die Öffnungen 43 in das Blutgefäß 10 gepresst werden.

Das Pumpengehäuse 36 ist dabei auf dem distalen Ende 44 des Hohlkatheters 45 abgestützt, wobei durch den Hohlkatheter 45 auch die Antriebswelle 46 verläuft, die an der Nabe 47 endet. Die Nabe 47 ist sinnvollerweise an beiden Enden des Pumpengehäuses 36 drehbar gelagert.

Ist der Betriebszustand durch völlige Entfaltung des Rotors bzw. der Förderschaufeln 37 und Aufpumpen des Pumpengehäuses 36 erreicht, so kann die Pumpe in diesem Zustand durch Härten sowohl des Pumpengehäuses als auch der Förderschaufeln stabilisiert werden. Dies geschieht beispielsweise durch Bestrahlung von außen mit UV-Licht, einer anderen Strahlung oder Ultraschall oder durch chemische Einwirkung entweder durch Zugabe eines geeigneten, eine Reaktion an den Förderelementen oder am Pumpengehäuse in Gang setzenden Stoffes oder durch Reaktion mit einem ohnehin im zu fördernden Blut befindlichen Stoff, der als Reaktionspartner oder Katalysator wirkt.

Auch hier kann alternativ dazu eine vorübergehende Versteifung oder Erhöhung einer Viskosität im Falle von Füllflüssigkeiten durch Anwendung von magnetischen oder elektrischen Feldern vorgesehen sein.

Entsprechende Felder können entweder von außerhalb des Patientenkörpers eingebracht bzw. eingestrahlt werden oder sie können durch entsprechende Sonden, die in die Nähe der Pumpe gebracht werden, appliziert werden.

Es ist auch für sämtliche in dieser Anmeldung dargestellten Ausführungsformen sowie auch davon unabhängig möglich, dass beispielsweise Elektromagnetische Strahlung wie zum Beispiel Licht, UV-Strahlung, Infrarotstrahlung, Kurzwellen oder Röntgenstrahlung zum Pumpenkopf geleitet wird, um dort eine Härtungsreaktion hervorzurufen. Dies kann zum Beispiel über einen geeigneten Lichtleiter, der beispielsweise durch den Hohlkatheter geführt werden kann, erfolgen.

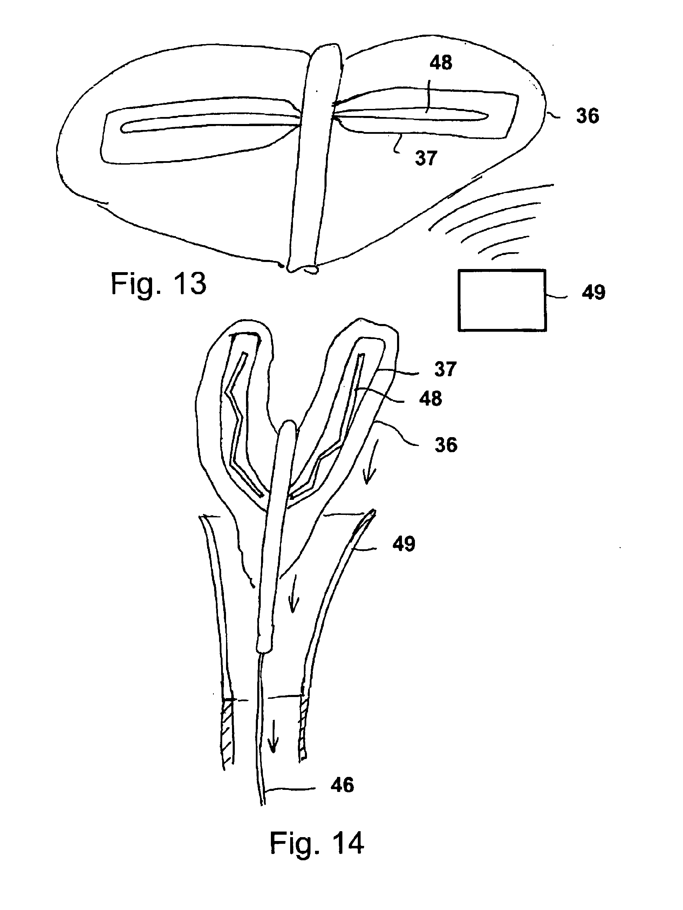

Fig. 13 zeigt schematisch einen Pumpenkopf mit einem Gehäuse 36 und Förderschaufeln 37, die Verstärkungsrippen 48 aufweisen. Diese sind typischerweise zur Stabilisierung des Betriebszustandes beispielsweise durch Vernetzung eines vernetzbaren Polymers versteift.

Um die Probleme zu lösen, die bei der Entnahme des Pumpenkopfes nach der Behandlung bei einem Patienten auftreten können, ist es notwendig, die Förderschaufeln 37 zu komprimieren. Dies kann beispielsweise dadurch geschehen, dass durch eine Strahlungsquelle 49 der Rotor weiter bestrahlt wird, so dass sich die Härtung durch weitere Vernetzung bis zur Versprödung fortsetzt. Sind die Versteifungsstege 48 versprödet, so können sie von selbst brechen oder einfach beim Entnehmen der Pumpe durch Zurückziehen des Pumpenkopfes in den Hohlkatheter 49 gebrochen werden.

Die Fig. 14 zeigt die Verstärkungsstege 48 innerhalb der Förderschaufeln 37 in geknicktem bzw. gebrochenem Zustand, wie der Pumpenkopf mitsamt dem Gehäuse 36 in das trichterförmige, distale Ende des Hohlkatheters 49 mittels der Antriebswelle 46 zurückgezogen wird. Das Zurückziehen kann jedoch auch durch andere Mittel wie beispielsweise parallel zu der Antriebswelle 46 in dem Hohlkatheter 49 verlaufende Züge erfolgen.

Auf die beschriebene Weise kann der Pumpenkopf ohne größeren mechanischen Widerstand in den Hohlkatheter eingezogen und zusammen mit diesem aus der Herzkammer bzw. durch das Blutgefäß hindurch aus dem Patientenkörper entfernt werden.

Alternativ zur Fortsetzung des Härtungsprozesses, der nach dem Transport zur Versteifung des Rotors genutzt wurde, bis zur Versprödung kann auch eine Behandlung erfolgen, die von der anfänglichen Versteifungsbehandlung unterschiedlich ist. Beispielsweise kann eine Versprödung oder ein Brechen durch Ultraschallbehandlung vorgesehen sein. Auch eine Temperaturabsenkung beispielsweise durch Einbringen eines Kühlmittels durch den Hohlkatheter 49 kann lokal bewerkstelligt werden, um den Rotor und/oder das Pumpengehäuse zu verspröden und bruchempfindlich zu machen. Am sinnvollsten ist dabei, lediglich den Rotor entsprechend zu brechen und das Pumpengehäuse intakt zu lassen, damit eventuell entstehende Bruchsplitter nicht in die Blutbahn gelangen können.

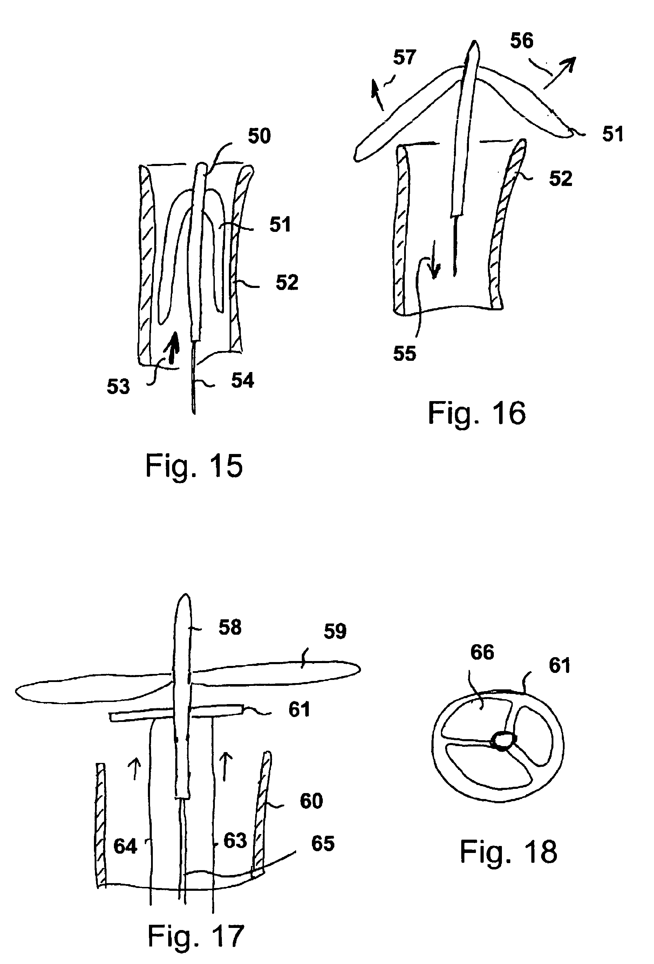

In den Fign. 15 und 16 ist ein möglicher Aufstellmechanismus für einen Rotor nach dem Transport und zum Übergang in den Betriebszustand dargestellt.

In Fig. 15 ist ein Rotor mit der Nabe 50 und den Förderschaufeln 51 im komprimierten Zustand innerhalb des Hohlkatheters 52 dargestellt, kurz bevor er in Richtung des Pfeils 53 aus dem Hohlkatheter innerhalb der Herzkammer herausgeschoben wird. Das Herausschieben kann mittels der Antriebswelle 54 oder weiterer nicht dargestellter Drähte bzw. Züge erfolgen. In diesem Zustand ist der Rotor noch ungehärtet und beweglich. Ist er aus dem Hohlkatheter herausgefahren, so ist es alternativ oder zusätzlich zu anderen Möglichkeiten der Expansion auch möglich, nachfolgend den Rotor ein Stück weit in Richtung des Pfeils 55 in Fig. 16 zurückzuziehen, so dass die Förderschaufeln 51 durch das Anstoßen am Rand des distalen Endes des Hohlkatheters 52 anstoßen und radial in Richtung der Pfeile 56, 57 aufgerichtet werden. Sodann kann ein Härten des Rotors oder ausschließlich der Förderschaufeln oder von Teilen der Förderschaufeln erfolgen, so dass der Rotor in expandierter Form stabilisiert ist. Daraufhin kann der Rotor wieder in Richtung des Pfeils 53 aus dem Hohlkatheter 52 heraus- und von diesem weggeschoben werden, um in die Betriebsposition zu gelangen. Bei der obigen Betrachtung ist das Gehäuse der Pumpe außer Betracht gelassen, dieses wird jedoch in der überwiegenden Zahl der Ausführungsformen zusätzlich vorgesehen sein und den Rotor umgeben.

Die Fig. 17 zeigt eine weitere Ausführungsform eines Rotors mit einer Nabe 58, an der Förderschaufeln 59 befestigt sind. Nach dem Ausfahren aus dem Hohlkatheter 60, ähnlich wie in der Fig. 15 dargestellt, können durch Nachschieben eines Aufrichtrades 61, das in der Fig. 18 in der Draufsicht deutlicher dargestellt ist, der Rotor bzw. die Förderschaufeln des Rotors aufgerichtet werden. Das Aufrichtrad 61 wird durch Schieben mittels mehrerer Schubelemente 63, 64 oder eines innerhalb des Hohlkatheters 60 verlaufenden schlauchartigen Elementes, das beispielsweise die Antriebswelle 65 umgeben kann, bewerkstelligt.

Im aufgerichteten Zustand des Rotors wird dieser dann gehärtet, und danach kann das Aufrichtrad 61 in den Hohlkatheter 60 zurückgezogen werden. Das Aufrichtrad 61 ist mit großen Durchgangsöffnungen 66 versehen, um die Strömungsverhältnisse der Pumpe nicht oder nur minimal zu beeinträchtigen.

In Ergänzung aller oben genannten Beispiele und auch als eigenständige Erfindung anwendbar ist es darüber hinaus auch möglich, den Vorgang des Härtens und/oder des Erweichens der Vorrichtung jeweils durch eine kurzzeitige Einwirkung eines Impulses, elektromagnetischen Feldes oder eines ähnlichen Effektes hervorzurufen, so dass die jeweilige Einwirkdauer auf das minimal notwendige Ausmaß beschränkt ist. So kann beispielsweise der Kristallisationsprozess der Flüssigkeit durch einen kurzen mechanischen Impuls ausgelöst werden, ähnlich dem Vorgehen bei sogenannten Wärmekissen. Das entsprechend kristallisierte Medium kann dann durch kurzzeitige lokale Wärmeeinwirkung wieder verflüssigt werden. Durch Versetzen des Mediums mit beispielsweise Metallpartikeln, die in einem entsprechenden Feld angeregt werden, könnte die Wärmeeinwirkung soweit lokal begrenzt werden, dass eine Schädigung des umgebenden Gewebes auf ein unschädliches Maß reduziert bzw. ganz vermieden wird.

Die erfindungsgemäße Vorrichtung und die erfindungsgemäßen Verfahren erlauben mit technisch übersichtlichem Aufwand die Beeinflussung der mechanischen Eigenschaften von Elementen einer in einen Patientenkörper eingebrachten Vorrichtung, speziell einer Blutpumpe, so dass diese in die geeignete Form für den Betrieb gebracht oder mit der erforderlichen Steifigkeit versehen werden kann, ohne dass die entsprechenden mechanischen Eigenschaften schon beim Einführen in den Patientenkörper vorhanden sein müssten. Dadurch werden neue Aufbauformen entsprechender Vorrichtungen/Pumpen möglich.

## Patentansprüche

1. Vorrichtung zur mechanischen Einwirkung auf ein Medium (30) mit wenigstens einem ersten Element (9, 9', 9'', 9''', 10, 10', 10'', 10''', 31, 37, 59), das durch Änderung wenigstens einer mechanischen Eigenschaft von einem Transportzustand in einen Betriebszustand gebracht werden kann, **dadurch gekennzeichnet, dass** das erste Element wenigstens teilweise aus einem Stoff (17, 18, 21, 22, 26, 27, 33, 36) besteht oder mit einem Stoff oder Stoffgemisch füllbar ist, der/das zum Übergang in den Betriebszustand eine Stoffumwandlung durch eine chemische Reaktion, insbesondere Vernetzung, oder einen Übergang vom flüssigen Zustand in einen festen Zustand durchläuft.

2. Vorrichtung nach Anspruch 1, **dadurch gekennzeichnet, dass** die Vorrichtung als Fluidpumpe (6) ausgebildet ist.

3. Vorrichtung nach Anspruch 2, **dadurch gekennzeichnet, dass** das erste Element (9, 9', 9'', 9''', 10, 10', 10'', 10''', 31, 37, 59) ein Teil eines Rotors, insbesondere ein Förderelement ist.

4. Vorrichtung nach Anspruch 3, **dadurch gekennzeichnet, dass** das erste Element (9, 9', 9'', 9''', 10, 10', 10'', 10''', 31, 37, 59) eine Förderschaufel einer Pumpe ist.

5. Vorrichtung nach Anspruch 2, **dadurch gekennzeichnet, dass** das erste Element ein Pumpengehäuse (7) ist.

6. Vorrichtung nach Anspruch 1 oder einem der folgenden, **dadurch gekennzeichnet, dass** die änderbare mechanische Eigenschaft die Steifigkeit und/oder die geometrische Form ist.

7. Vorrichtung nach Anspruch 1 oder einem der folgenden, **dadurch gekennzeichnet, dass** das erste Element (9, 9', 9'', 9''', 10, 10', 10'', 10''', 31, 37, 59) ein härtbares Material aufweist.

8. Vorrichtung nach Anspruch 7, **dadurch gekennzeichnet, dass** das Material durch Temperatureinwirkung, elektrische und/oder magnetische Felder, Strahlung, mechanische Einwirkung oder Hinzufügung eines weiteren Stoffs oder Kontakt mit einem weiteren Stoff oder durch Initialisierung einer Kristallisation härtbar ist.

9. Vorrichtung nach Anspruch 8, **dadurch gekennzeichnet, dass** der weitere Stoff in dem Medium enthalten ist, auf das die Vorrichtung einwirkt.

10. Vorrichtung nach Anspruch 1 oder einem der folgenden, **dadurch gekennzeichnet, dass** durch eine fortgesetzte oder weitere Reaktion das erste Element reversibel oder irreversibel in einen Zustand überführbar ist, in dem die Vorrichtung transportierbar ist oder in dem es durch mechanisches Zerbrechen in einen Transportzustand gebracht werden kann.

11. Verfahren zum Betrieb einer Vorrichtung gemäß Anspruch 1 oder einem der folgenden, **dadurch gekennzeichnet, dass** die Vorrichtung zunächst in einen Körper eines Lebewesens eingeführt wird, dass darauf der Stoff / das Stoffgemisch des ersten Elementes eine Reaktion, insbesondere Vernetzung, oder eine Kristallisation erfährt und dass die Vorrichtung darauf in Gang gesetzt wird.

12. Verfahren nach Anspruch 11, **dadurch gekennzeichnet, dass** vor der Reaktion oder Kristallisation der oder ein Stoff in wenigstens einen Hohlraum des ersten Elementes eingeführt wird.

13. Verfahren nach Anspruch 11 oder 12, **dadurch gekennzeichnet, dass** zur Initiierung, Unterstützung oder Durchführung der Reaktion oder Kristallisation auf die Vorrichtung durch Strahlung, Temperaturveränderung oder mechanische Einwirkung, insbesondere Ultraschall, oder durch elektrische und/oder magnetische Felder eingewirkt wird.

14. Vorrichtung zur mechanischen Einwirkung auf ein Medium mit wenigstens einem ersten Element (9, 9', 9'', 9''', 10, 10', 10'', 10''', 31, 37, 59), das durch Änderung von wenigstens einer mechanischen Eigenschaft von einem Transportzustand in einen Betriebszustand gebracht werden kann, **dadurch gekennzeichnet, dass** das erste Element wenigstens teilweise aus einem Stoff (17, 18, 21, 22, 26, 27, 33, 36) besteht oder mit einem Stoff oder Stoffgemisch füllbar ist, der/das, solange er einer Bestrahlung, einem elektrischen und/oder einem magnetischen Feld ausgesetzt ist, gegenüber dem Zustand ohne derartige Einwirkung geänderte mechanische Eigenschaften, insbesondere geänderte Steifigkeit/ Viskosität oder Form aufweist.

15. Verfahren zum Betrieb einer Vorrichtung gemäß Anspruch 14, **dadurch gekennzeichnet, dass** die Vorrichtung nach ihrer Einführung in den Körper eines Lebewesens der entsprechenden Einwirkung ausgesetzt und währenddessen in Gang gesetzt wird oder dass die Vorrichtung während des Transports einer entsprechenden Einwirkung ausgesetzt wird, die zum Übergang in den Betriebszustand geändert oder beendet wird.
